(19) Europäisches Patentamt
European Patent Office
Office européen des brevets

(11) Veröffentlichungsnummer: **0 259 882 B1**

# (12) EUROPÄISCHE PATENTSCHRIFT

(45) Veröffentlichungstag der Patentschrift: **23.11.89**

(51) Int. Cl.⁴: **C07B 45/06**

(21) Anmeldenummer: **87113279.1**

(22) Anmeldetag: **11.09.87**

## (54) Verfahren zur Reduktion organischer Schwefelverbindungen.

(30) Priorität: **12.09.86 DE 3631073**

(43) Veröffentlichungstag der Anmeldung: **16.03.88 Patentblatt 88/11**

(45) Bekanntmachung des Hinweises auf die Patenterteilung: **23.11.89 Patentblatt 89/47**

(84) Benannte Vertragsstaaten: **BE CH DE FR GB IT LI NL**

(56) Entgegenhaltungen:
**DE-A- 1 643 385**
**DE-A- 1 903 968**

**K. FUJIMOTO ET AL., NIPPON KAGAKU KAISHI 1976, 1062**

(73) Patentinhaber: **BASF Aktiengesellschaft, Carl-Bosch-Strasse 38, D-6700 Ludwigshafen(DE)**

(72) Erfinder: **Dockner, Toni, Dr., Grossgasse 6, D-6701 Meckenheim(DE)**
Erfinder: **Sauerwald, Manfred, Dr., Gebhardtstrasse 16, D-6701 Roedersheim-Gronau(DE)**

**EP 0 259 882 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Reduktion von Sulfonsäuren, Sulfonsäurehalogeniden, -estern, -amiden, -anhydriden, Sulfonen, Sulfoxiden und/oder Disulfanen.

In Houben-Weyl, Methoden der organischen Chemie, Bd. 9, S. 23–33, 1955; Bd. 4/1c, S. 486–489 und 664–667, 1980, Bd. 4/1d, S. 680 und 681, 1981 sowie Bd. E 11/1, S. 48–54, 1985, sind zahlreiche Methoden zur Reduktion organischer Schwefelverbindungen beschrieben. Hier ist insbesondere die katalytische Hydrierung in Gegenwart von Metallkatalysatoren zu nennen, die insofern problematisch ist, als Sulfane, Disulfane, Mercaptane und Thiophenole starke Katalysatorgifte darstellen, so daß lange Reaktionszeiten oder der Einsatz hoher Katalysatormengen erforderlich ist.

Brauchbare Katalysatoren zur Reduktion von aromatischen Sulfonsäurederivaten sind Sulfide von Cobalt, Molybdän, Eisen oder Nickel (siehe Ullmanns Encyklopädie der technischen Chemie, 4. Aufl., Bd. 23, S. 175–215, insb. S. 188, Weinheim, 1983). Für die Durchführung der Reduktion sind jedoch Drücke von zirka 100 bar notwendig, was einen hohen apparativen Aufwand erfordert. Um bei der Reduktion von Sulfonsäurechloriden Ausbeuteverluste infolge partieller Hydrolyse durch Reaktionswasser zu vermeiden, muß in Gegenwart von Basen wie Calciumoxid oder Magnesiumoxid gearbeitet werden. Die weniger reaktiven Sulfonsäuren können nach diesen Verfahren nicht reduziert werden. Bei Temperaturen von 100–200°C werden als Reaktionsprodukte die entsprechenden Mercaptane bzw. Thiophenole gebildet, weitere Temperaturerhöhung führt zu Kohlenwasserstoffen (K. Itabashi, Yuki Gosei Kagaku Kyokai Shi 19, 601, 1961; K. Itabashi, Yuki Gosei Kagaku Kyokai Shi 19, 266 u. 271, 1961).

Neben der katalytischen Hydrierung kommt bei der Herstellung von Thiolen aus Sulfonsäurechloriden und Disulfanen die Reduktion mit Eisen, Zink oder Zinn in Eisessig, Salzsäure oder Schwefelsäure in Betracht. Die Problematik dieses Verfahrens liegt in dem Anfall größerer Mengen ökologisch nicht unbedenklicher Metallsalze, die entsprechend aufgearbeitet werden müssen. Nachteilig ist ferner, daß lediglich die reaktiven Derivate der Sulfonsäuren, nicht aber die Sulfonsäuren selbst, reduziert werden können.

Neben den genannten Verfahren stehen eine Reihe weiterer Methoden zur Verfügung; eine Übersicht über die Reduktion von Sulfonen und Sulfoxiden geben z.B. J. Drabowicz, T. Numata und S. Oae in Org. Prep. Proc. Int. 9, 64, 1977. Diese Methoden sind jedoch alle für Reaktionen im technischen Maßstab wenig geeignet, da entweder umweltbelastende Nebenprodukte wie Metallsalze oder Abwässer, die mit Salzen oder Schwefelverbindungen verunreinigt sind, anfallen oder andererseits teure und schwierig zu handhabende Reduktionsmittel wie z.B. Lithiumaluminiumhydrid verwendet werden.

Aus dem Stand der Technik ist ferner bekannt, daß Schwefeldioxid mit Isopentan bei 400 bis 500°C an aktivem Kohlenstoff zu Schwefelwasserstoff und Schwefel umgesetzt werden kann (K. Fujimoto, K. Masamizu, S. Asaoka, T. Kunugi, Nippon Kagaku Kaishi 1976, 1062).

Der Erfindung lag nun die Aufgabe zugrunde, ein Verfahren zur Reduktion von organischen Sulfonsäuren, Sulfonsäurederivaten, Sulfonen, Sulfoxiden und Disulfanen zu finden, das breit anwendbar ist, die geschilderten Nachteile bekannter Verfahren überwindet, billig ist, sich durch hohe Selektivitäten auszeichnet und vor allem im großtechnischen Betrieb anwendbar ist.

Demgemäß wurde ein Verfahren zur Reduktion von Sulfonsäuren, Sulfonsäurehalogeniden,-estern, amiden, -anhydriden, Sulfonen, Sulfoxiden und/oder Disulfanen gefunden, das dadurch gekennzeichnet ist, daß man die Schwefelverbindungen in der Flüssig- oder Gasphase mit Kohlenwasserstoffen in Gegenwart von Kohlenstoff bei Temperaturen von 100 bis 500°C umsetzt.

Nach dem erfindungsgemäßen Verfahren werden aliphatische, cycloaliphatische, aromatische und araliphatische Disulfane, Sulfonsäuren und Sulfonsäurederivate, wie Halogenide, z.B. Chloride oder Bromide, Anhydride, Ester und Amide, gemäß den allgemeinen Gleichungen a) und b) zu Thiolen reduziert. Die Reduktion aliphatischer, aromatischer und araliphatischer Sulfone und Sulfoxide verläuft entsprechend Gleichung c) bis zum Sulfan:

Allgemeine Reaktionsgleichungen, in denen R und R′ z.B. einen Alkyl-, Aryl- oder Aralkylrest bedeuten:

a) $$R-SO_2-X + 6[H] \longrightarrow R-SH + 2H_2O + HX$$

$X$ = Halogen, $R'SO_3$, $R'O$, $NH_2$, $OH$

b) $$R-S-S-R' + 2[H] \longrightarrow R-SH + R'-SH$$

c) $$R-SO_n-R' + 2n[H] \longrightarrow R-S-R' + nH_2O$$

$n$ = 1 oder 2

Der für die Reduktion benötigte Wasserstoff entstammt dem Kohlenwasserstoff, der dabei in wasserstoffärmere Derivate bis hin zum Kohlenstoff übergeht.

Es wurde überraschend gefunden, daß bei Temperaturen von 100 - 500°C mit einfachen paraffinischen, olefinischen und/oder aromatischen Kohlenwasserstoffen sowie deren Gemischen als Wasserstoffüberträger eine sehr selektive Reduktion der genannten Schwefelverbindungen möglich ist.

Als Kohlenwasserstoffe werden beispielsweise hochsiedende Mineralölfraktionen verwendet, deren Siedebereiche höher liegen als die Reaktionstemperatur, die 100 bis 500°C, vorzugsweise 200 - 400°C, insbesondere 250 - 350°C beträgt. Solche Kohlenwasserstoffe sind z.B. technisches Weißöl, Vakuumgasöl, Heizöl S, Vakuumrückstandsöl, geschmolzenes Paraffinwachs oder aromatisches Kohlenwasserstofföl. Ebenfalls geeignet sind partiell hydrierte Aromaten, wie etwa 1,2,3,4-Tetrahydronaphthalin oder 9,10-Dihydroanthracen. Es ist aber auch möglich, niedriger siedende Kohlenwasserstoffe bzw. Kohlenwasserstoffgemische, wie z.B. Methan, Ethan, Acetylen, Propan, Propen, Butan, Buten, Pentan, Penten, Cyclopentan, Hexan, Hexen, Cyclohexan usw., Heizöl L, Benzin, Leichtbenzin oder Flüssiggas einzusetzen, wobei man sowohl unter erhöhtem Druck in der Flüssigphase als auch in der Gasphase arbeiten kann.

Die Kohlenwasserstoffe können vorteilhaft in einer Menge von 10 bis 1000 g, inbesondere 50 bis 500 g pro Mol Ausgangsstoff eingesetzt werden.

Die Reduktion wird erfindungsgemäß in Gegenwart von elementarem Kohlenstoff durchgeführt. Dieser Kohlenstoff kann entweder von vornherein als Bestandteil des verwendeten Kohlenwasserstoffs vorhanden sein, während der Reaktion entstehen oder zugesetzt werden. Geeignete Kohlenstoffzusätze sind z.B. Petrolkoks, Ruß oder eine andere Form des Graphits. Besonders geeignet sind Aktivkohlen wie Carboraffin P® oder Tierkohle, die z.B. mit Zinkchlorid, Phosphorsäure oder Wasserstoff aktiviert sind.

Die Anwesenheit von elementarem Kohlenstoff im Reaktionsgemisch beschleunigt die Reaktion und steigert den Umsatz. Vorzugsweise liegen in dem Reaktionsgemisch 1 bis 50, insbesondere 5 bis 25 Gew.% Kohlenstoff, bezogen auf den Kohlenwasserstoff, vor.

Als Ausgangsstoff für die Herstellung von Thiolen können vorteilhaft Sulfonsäurehalogenide, -anhydride, -ester und -amide verwendet werden. Ein besonderer Vorteil des Verfahrens besteht aber darin, daß selbst die weniger reaktionsfähigen Sulfonsäuren unter den Reaktionsbedingungen zu Thiolen reduziert werden. Die Reaktion ist auf aliphatische, cycloaliphatische, araliphatische und aromatische Sulfonsäuren bzw. deren Derivate anwendbar, doch besitzt sie insbesondere bei den aromatischen Vertretern präparative Bedeutung, da die entsprechenden Vorstufen leicht durch Sulfonierungsreaktionen zugänglich sind. Diesbezüglich besteht ein weiterer Vorteil darin, daß nach dem hier gefundenen Verfahren auch aromatische Dithiole gut zugänglich sind, was bei anderen bekannten Methoden aufgrund von Nebenreaktionen wie z.B. Polymerisation häufig zu Problemen führt (vgl. C.M. Suter, P.H. Scrutchfield, J. Am. Chem. Soc. 58, 54, 1936).

Das erfindungsgemäße Verfahren ist insbesondere auch zur Herstellung von Thiolen aus Disulfanen geeignet, wobei es sich sowohl um symmetrische als auch um unsymmetrische Disulfane mit aliphatischen, cycloaliphatischen, araliphatischen oder aromatischen Resten handeln kann. Aliphatische Disulfane sind z.B. leicht durch nukleophile Substitution aus Alkylhalogeniden und Metalldisulfiden zugänglich. Ferner lassen sich durch Umsetzung von symmetrischen oder unsymmetrischen aliphatischen, araliphatischen oder aromatischen Sulfoxiden und Sulfonen die entsprechenden Sulfane herstellen.

Die organischen Reste der Sulfonsäuren, Sulfonsäurederivate, Disulfane, Sulfoxide und Sulfone sind breit variierbar und unterliegen keinen besonderen Einschränkungen. Aliphatische, cycloaliphatische, araliphatische und/oder aromatische Reste sind z.B. Alkyl-, Cycloalkyl-, Alkenyl-, Alkinyl-, Aralkyl- oder Arylreste, die unter den Reaktionsbedingungen inerte Substituenten tragen können.

Alkylreste sind z.B. verzweigte oder unverzweigte Reste mit 1 bis 20, insbesondere 1 bis 12 Kohlenstoffatomen, z.B. Methyl-, Ethyl-, n-Propyl-, i-Propyl-, n-Butyl-, i-Butyl-, tert.-Butyl-, Pentyl-, Hexyl-, Octyl- oder Dodecylreste. Cycloalkylreste sind z.B. solche mit 5 bis 8 Ringgliedern, insbesondere Cyclopentyl- und Cyclohexylreste. Die zu reduzierende Gruppe kann, wie z.B. beim Sulfolan, selbst ein Teil des Ringes sein. Die Alkylreste können weiterhin Doppel- und/oder Dreifachbindungen enthalten, wobei α,β-ungesättigte Sulfonsäuren und deren Derivate sowie α,β-ungesättigte Sulfone und Sulfoxide allerdings zu den entsprechenden gesättigten Thiolen bzw. Sulfanen reduziert werden. Außerdem können die Alkylreste unter den Reaktionsbedingungen inerte funktionelle Gruppen wie Alkoxy-, Carboxyl-, Nitril-, Amino- oder Carbonylgruppen tragen.

Die in Frage kommenden Arylreste leiten sich z.B. von den Grundkörpern Benzol, Diphenyl, Naphthalin, Anthracen usw. ab. Diese können zusätzlich noch durch inerte Gruppen wie Halogen-, z.B. Fluor, Chlor oder Brom, Alkyl-, Alkoxy-, Carboxyl-, Nitril-, Carbonyl- oder Aminogruppen substituiert sein. Ferner kann es sich bei den Arylresten um heteroaromatische Gruppen handeln, die ein oder mehrere Stickstoff-, Sauerstoff- oder Schwefelatome sowie deren Kombinationen enthalten. Hier sind beispielsweise Heteroaromaten wie Pyrrol, Pyrazol, Imidazol, Pyridin, Pyridazin, Pyrimidin, Pyrazin, Chinolin, iso-Chinolin, Chinoxalin, Indol, Furan, Oxazol, Isoxazol, Thiophen und Thiazol zu nennen.

Araliphatische Reste sind z.B. solche, die sich aus den genannten aliphatischen und aromatischen Resten zusammensetzen. Dabei kann sich die zu reduzierende Schwefelgruppe sowohl im aliphatischen als auch im aromatischen Teil befinden. Beispielsweise seien der Benzyl-, der Phenylethyl- oder der o-Tolylrest genannt.

Bei der Durchführung in flüssiger Phase sollte mindestens der Hauptteil der reagierenden Kohlenwasserstoffe flüssig vorliegen. Die flüssige Phase enthält suspendierten Kohlenstoff, der entweder während der Reaktion entsteht, zugesetzt wird oder als Bestandteil der verwendeten Kohlenwasserstoffe, z.B. in Heizöl S oder Vakuumrückstandsöl, vorhanden ist. Wenn der Kohlenwasserstoff nicht von vornherein Kohlenstoff enthält, kann die Umsetzung durch Zugabe von elementarem Kohlenstoff zum Reaktionsgemisch beschleunigt werden.

Die Umsetzung in der Flüssigphase wird zweckmäßig so ausgeführt, daß die zu reduzierende Schwefelverbindung fest, flüssig oder gasförmig, gegebenenfalls zusammen mit einem Inertgas wie z.B. Stickstoff in eine auf Reaktionstemperatur erhitzte Suspension von Kohlenstoff in einem Kohlenwasserstoff eingebracht wird. Je nach Reaktionstemperatur und Siedepunkt verlassen die Reaktionsprodukte unmittelbar nach der Umsetzung das Reaktionsgefäß und werden durch Abkühlung kondensiert, wonach sie nach bekannten Methoden, z.B. destillativ, aufgearbeitet werden können. Produkte mit höherem Siedepunkt verbleiben in dem Reaktionsgemisch und können nach der Umsetzung in an sich bekannter Weise beispielsweise durch Destillation oder Extraktion isoliert werden.

Bei der Durchführung in der Gasphase wird die zu reduzierende Verbindung gasförmig zusammen mit einem Kohlenwasserstoff über elementaren Kohlenstoff als Katalysator geleitet.

Die Umsetzung kann sowohl diskontinuierlich als auch kontinuierlich bei Normaldruck, erhöhtem oder vermindertem Druck in den üblicherweise verwendeten Reaktoren, z.B. in Rührkesseln oder in zylindrischen Reaktoren, durchgeführt werden.

Das erfindungsgemäße Verfahren macht es auf eine billige, technisch einfache Art möglich, organische Schwefelverbindungen wie Sulfonsäuren, Sulfonsäurederivate, Sulfone, Sulfoxide und Disulfane unter Wasserstoffübertragung zu reduzieren, ohne daß es zum Anfall von die Umwelt belastenden Metallsalzen oder salzhaltigen Abwässern kommt.

Die folgenden Beispiele sollen das Verfahren verdeutlichen, aber nicht einschränken.

<u>Beispiele 1 bis 6</u>

Reduktion von aromatischen Sulfonsäuren, Sulfonsäurederivaten und Diphenyldisulfan

450 g technisches Weißöl und 50 g Aktivkohle (Carboraffin P®) wurden auf 350°C erhitzt und stündlich mit 0,3 mol des in nachfolgender Tabelle angegebenen Ausgangsstoffes zusammen mit 30 l Stickstoff versetzt. Dabei wurde der Ausgangsstoff auf die Oberfläche des gerührten Weißöl/Kohle-Gemisches getropft und die den Reaktionskolben verlassenden Dämpfe kondensiert. Feste Ausgangsstoffe wurden zuvor bei 90 bzw. 140°C aufgeschmolzen.

Die nach einer Versuchsdauer von 4 Stunden im Kondensat anfallenden Produktmengen und Ausbeuten sind der Tabelle zu entnehmen. Im Fall von Toluolsulfonsäurechlorid als Ausgangsstoff wurde das Kondensat weiter aufgearbeitet, indem man es mit einer 20%igen wäßrigen Natronlauge behandelte, die wäßrige Phase ansäuerte und mit Methylenchlorid extrahierte. Nach Abtrennen des Methylenchlorids erhielt man 131,4 g p-Thiokresol, entsprechend einer Ausbeute von 88,3 %, bezogen auf p-Toluolsulfonsäurechlorid.

Tabelle

| Beispiel | Ausgangsstoff | Menge/h g | Produkt | Menge[a] g | Ausbeute[b] % |
|---|---|---|---|---|---|
| 1 | p-$CH_3C_6H_4SO_2Cl$ | 57 | p-$CH_3$-$C_6H_4SH$ | 131,4 | 88,3 |
| 2 | p-$CH_3C_6H_4SO_3H \cdot H_2O$ | 57 | p-$CH_3$-$C_6H_4SH$ | 124,0 | 83,3 |
| 3 | p-$CH_3C_6H_4SO_3CH_3$ | 56 | p-$CH_3$-$C_6H_4SH$ | 91,4 | 61,2 |
| 4 | $C_6H_5SO_2Cl$ | 53 | $C_6H_5SH$ | 106,8 | 80,8 |
| 5 | $C_6H_5SO_3H$ | 48 | $C_6H_5SH$ | 117,1 | 87,6 |
| 6 | $C_6H_5$-S-S-$C_6H_5$ | 66 | $C_6H_5SH$ | 263,2 | 98,8 |

[a] die nach einer Versuchsdauer von 4 Stunden im Kondensat anfallende Produktmenge wurde in den Beispielen 2 bis 6 durch gaschromatographische Analyse ermittelt.
[b] bezogen auf eingesetzten Ausgangsstoff.

Beispiel 7

Reduktion von Di-n-butyldisulfan

Wie in den Beispielen 1 bis 6 beschrieben, wurden stündlich 54 g (0,3 mol) Di-n-butyldisulfan (bei 90°C aufgeschmolzen) umgesetzt.
Nach einer Versuchsdauer von vier Stunden erhielt man ein Kondensat, das laut gaschromatographischer Analyse neben 54,4 g nicht umgesetztem Ausgangsmaterial 134,5 g 1-Butanthiol enthielt. Demnach betrug der Umsatz 74,8 % und die Selektivität 82,3 %.

Beispiel 8

Reduktion von Methansulfonsäure

Wie in den Beispielen 1 bis 6 beschrieben, wurden stündlich 29 g (0,3 mol) Methansulfonsäure umgesetzt. Die den Reaktor gasförmig verlassenden Produkte wurden zunächst bei 0°C, dann bei -78°C kondensiert und die anfallenden Kondensate gaschromatographisch untersucht. Nach einer Versuchsdauer von vier Stunden erhielt man neben 17,4 g nicht umgesetzter Methansulfonsäure 25,3 g Methanthiol. Der Umsatz betrug demnach 85,0 % und die Selektivität 51,5 %.

Beispiel 9

Reduktion von Diphenylsulfoxid

Wie in den Beispielen 1 bis 6 beschrieben, wurden stündlich 60 g (0,3 mol) Diphenylsulfoxid (bei 90°C aufgeschmolzen) umgesetzt.
Nach einer Versuchsdauer von vier Stunden erhielt man ein Kondensat, das laut gaschromatographischer Analyse 189,4 g Diphenylsulfan enthielt. Dies entspricht einer Ausbeute von 85,7 %.

Beispiel 10

Reduktion von Dimethylsulfoxid

Wie in den Beispielen 1 bis 6 beschrieben, wurden stündlich 23 g (0,3 mol) Dimethylsulfoxid umgesetzt.
Nach einer Versuchsdauer von vier Stunden erhielt man ein Kondensat, das laut gaschromatographischer Analyse 4,4 g nicht umgesetztes Dimethylsulfoxid und 45,2 g Dimethylsulfan enthielt. Der Umsatz betrug demnach 95,2 % und die Selektivität 64,9 %.

## Patentansprüche

1. Verfahren zur Reduktion von Sulfonsäuren, Sulfonsäurehalogeniden, -estern, -amiden, -anhydriden, Sulfonen, Sulfoxiden und/oder Disulfanen zu den entsprechenden Thiolen oder Sulfanen, dadurch gekennzeichnet, daß man die zu reduzierenden Schwefelverbindungen in der Flüssig- oder Gasphase mit Kohlenwasserstoffen in Gegenwart von Kohlenstoff bei Temperaturen von 100 bis 500°C umsetzt.
2. Verfahren gemäß Anspruch 1, dadurch gekennzeichnet, daß man als Kohlenwasserstoff hochsiedende Mineralöle verwendet, deren Siedepunkte höher liegen als die Reaktionstemperatur.
3. Verfahren gemäß den Ansprüchen 1 und 2, dadurch gekennzeichnet, daß man als Kohlenwasserstoff Vakuumrückstand, Heizöl S oder technisches Weißöl verwendet.

4. Verfahren gemäß Anspruch 1, dadurch gekennzeichnet, daß man niedrigsiedende Kohlenwasserstoffe wie Heizöl L, Benzin, Leichtbenzin oder niedere Kohlenwasserstoffe wie Methan, Ethan, Propan oder Butan verwendet und die Umsetzung in der Gasphase oder unter erhöhtem Druck in der Flüssigphase durchführt.

5. Verfahren gemäß den Ansprüchen 1 bis 4, dadurch gekennzeichnet, daß man den Kohlenstoff in einer Menge von 1 bis 50 Gew.-%, bezogen auf den Kohlenwasserstoff, verwendet.

6. Verfahren gemäß den Ansprüchen 1 bis 5, dadurch gekennzeichnet, daß man als Kohlenstoff Aktivkohle verwendet.

7. Verfahren gemäß den Ansprüchen 1 bis 6, dadurch gekennzeichnet, daß man Sulfonsäuren und deren Derivate zu Thiolen reduziert.

8. Verfahren gemäß den Ansprüchen 1 bis 6, dadurch gekennzeichnet, daß man Disulfane zu Thiolen reduziert.

9. Verfahren gemäß den Ansprüchen 1 bis 6, dadurch gekennzeichnet, daß man Sulfone oder Sulfoxide zu Sulfanen reduziert.

## Revendications

1. Procédé de réduction des acides sulfoniques, des halogénures, esters, amides et anhydrides d'acides sulfoniques, des sulfones, des sulfoxydes et/ou des disulfanes en thiols ou sulfanes correspondants, caractérisé en ce qu'on met en réaction les composés soufrés à réduire, en phase liquide ou gazeuse, avec des hydrocarbures en présence de carbone à des températures de 100 à 500°C.

2. Procédé selon la revendication 1, caractérisé en ce qu'on utilise comme hydrocarbure une huile minérale de haut point d'ébullition, qui est supérieur à la température de réaction.

3. Procédé selon la revendication 1 ou 2, caractérisé en ce qu'on utilise comme hydrocarbure un résidu court de distillation poussée, de l'huile lourde S ou de l'huile blanche technique.

4. Procédé selon la revendication 1, caractérisé en ce qu'on utilise des hydrocarbures à bas point d'ébullition comme l'huile lourde L, l'essence légère ou des hydrocarbures inférieurs, comme le méthane, l'éthane, le propane ou le butane et on effectue la réaction en phase gazeuse, ou en phase liquide sous pression élevée.

5. Procédé selon les revendications 1 à 4, caractérisé en ce qu'on utilise le carbone à raison de 1 à 50% en poids par rapport à l'hydrocarbure.

6. Procédé selon les revendications 1 à 5, caractérisé en ce qu'on utilise comme carbone du charbon actif.

7. Procédé selon les revendications 1 à 6, caractérisé en ce qu'on réduit en thiols des acides sulfoniques et leurs dérivés.

8. Procédé selon les revendications 1 à 6, caractérisé en ce qu'on réduit en thiols des disulfanes.

9. Procédé selon les revendications 1 à 6, caractérisé en ce qu'on réduit en sulfanes des sulfones ou des sulfoxydes.

## Claims

1. A process for the reduction of a sulfonic acid, sulfonyl halide, sulfonate, sulfonamide, sulfonic acid anhydride, sulfone, sulfoxide or disulfane to the corresponding thiol or sulfane, wherein the sulfur compound to be reduced is reacted in the liquid or gas phase with a hydrocarbon in the presence of carbon at from 100 to 500°C.

2. A process as claimed in claim 1, wherein the hydrocarbon used is a high boiling mineral oil whose boiling point is higher than the reaction temperature.

3. A process as claimed in either of claims 1 and 2, wherein the hydrocarbon used is a vacuum residue, heavy fuel oil or technical grade white oil.

4. A process as claimed in claim 1, wherein a low boiling hydrocarbon, such as light fuel oil, gasoline, naphtha or a lower hydrocarbon, such as methane, ethane, propane or butane is used and the reaction is carried out in the gas phase or under superatmospheric pressure in the liquid phase.

5. A process as claimed in any of claims 1 to 4, wherein the carbon is used in an amount of from 1 to 50% by weight, based on the hydrocarbon.

6. A process as claimed in any of claims 1 to 5, wherein the carbon used is active carbon.

7. A process as claimed in any of claims 1 to 6, wherein a sulfonic acid or its derivative is reduced to a thiol.

8. A process as claimed in any of claims 1 to 6, wherein a disulfane is reduced to a thiol.

9. A process as claimed in any of claims 1 to 6, wherein a sulfone or sulfoxide is reduced to a sulfane.